(19) Europäisches Patentamt

European Patent Office

Office européen des brevets

(11) **EP 0 944 677 B1**

(12) **EUROPÄISCHE PATENTSCHRIFT**

(45) Veröffentlichungstag und Bekanntmachung des Hinweises auf die Patenterteilung:
**02.04.2003   Patentblatt 2003/14**

(51) Int Cl.[7]: **C09C 1/00**, C09D 7/12, C09D 11/00, C08K 3/00, C04B 33/14, A61K 7/00, C03C 4/02

(21) Anmeldenummer: **97927029.5**

(22) Anmeldetag: **23.05.1997**

(86) Internationale Anmeldenummer:
**PCT/EP97/02652**

(87) Internationale Veröffentlichungsnummer:
**WO 98/053012 (26.11.1998 Gazette 1998/47)**

(54) **MEHRSCHICHTIGE INTERFERENZPIGMENTE**

MULTI-COATED INTERFERENCE PIGMENTS

PIGMENTS DE POLARISATION MULTICOUCHES

(84) Benannte Vertragsstaaten:
**DE ES FI FR GB IT**

(43) Veröffentlichungstag der Anmeldung:
**29.09.1999   Patentblatt 1999/39**

(73) Patentinhaber: **MERCK PATENT GmbH**
**64271 Darmstadt (DE)**

(72) Erfinder:
• **ANDES, Stephanie**
  **D-63477 Maintal 3 (DE)**
• **BAUER, Gerd**
  **D-63801 Kleinostheim (DE)**
• **BRENNER, Günter**
  **D-64347 Griesheim (DE)**
• **BRÜCKNER, Dieter**
  **D-64289 Darmstadt (DE)**
• **SCHMELZ, Michael**
  **D-65830 Kriftel (DE)**
• **HEYLAND, Andrea**
  **D-64385 Ober-Kainsbach (DE)**
• **KUNTZ, Matthias**
  **D-64342 Seeheim (DE)**
• **OSTERRIED, Karl**
  **D-64807 Dieburg (DE)**
• **PFAFF, Gerhard**
  **D-64839 Münster (DE)**

(56) Entgegenhaltungen:
**US-A- 3 395 203          US-A- 3 767 443**
**US-A- 4 168 986**

**Beschreibung**

Die Erfindung betrifft mehrschichtige Interferenzpigmente mit plättchenförmigem Titandioxid als Substrat.

Mehrschichtige Pigmente mit niedriger Transparenz sind bekannt. Die Metalloxidschichten werden entweder im Naßverfahren durch Auffällen der Metalloxidhydrate aus einer Metallsalzlösung auf ein Trägermaterial oder durch Aufdampfen oder Sputtern im Vakuum hergestellt. Generell sind die Aufdampfverfahren für eine Massenproduktion von Pigmenten aufwendig und kostspielig. So beschreibt US 4 434 010 ein mehrschichtiges Interferenzpigment, bestehend aus einer zentralen Schicht eines reflektierenden Materials (Aluminium) und alternierenden Schichten zweier transparenter, dielektrischer Materialien mit hoher und niedriger Brechzahl, beispielsweise Titandioxid und Siliziumdioxid beiderseits der zentralen Aluminiumschicht. Dieses Pigment wird für den Druck von Wertpapieren eingesetzt.

JP H7-759 (Kokoku) beschreibt ein mehrschichtiges Interferenzpigment mit metallischem Glanz. Es besteht aus einem Substrat, das mit alternierenden Schichten von Titandioxid und Siliziumdioxid beschichtet ist. Das Substrat wird aus Aluminium-, Gold- oder Silberplättchen oder Plättchen aus Glimmer und Glas, die mit Metallen beschichtet sind, gebildet. Es handelt sich demnach um ein typisches Metalleffektpigment. Dieses Pigment besitzt hohes Deckvermögen. Für Anwendungen, bei denen eine hohe Transparenz des pigmentierten Materials gefordert wird, beispielsweise für Agrarfolien, ist das Pigment ungeeignet. Weiterhin hat es den Nachteil, daß der typische Tiefeneffekt von Interferenzpigmenten nicht erzeugt wird, da durch die Totalreflextion des Lichtes an der den Kern bildenden Metallschicht, mehrere Pigmentteilchen nicht in Wechselwirkung treten können. Der Interferenzeffekt bleibt deshalb auf die, sich auf der Metallschicht befindlichen Schichten begrenzt.

Glimmer ist das Substrat, das am häufigsten für die Herstellung von Interferenzpigmenten eingesetzt wird.

Glimmerpigmente werden in großem Umfang in der Druck- und Lackindustrie, in der Kosmetik und in der Kunststoffverarbeitung verwendet. Sie zeichnen sich durch Interferenzfarben und einen hohen Glanz aus. Für die Ausbildung extrem dünner Schichten sind aber Glimmerpigmente nicht geeignet, weil Glimmer als Substrat für die Metalloxidschichten des Pigmentes bereits eine Dicke von 200 bis 1200 nm aufweist. Nachteilig ist weiterhin, daß die Dicke der Glimmerplättchen, teilweise deutlich um einen Mittelwert schwankt. Außerdem handelt es sich bei Glimmer um ein natürlich vorkommendes Mineral, das durch Fremdionen verunreinigt ist. Desweiteren sind technisch sehr aufwendige und zeitraubende Aufbereitungsschritte notwendig. Hierzu zählen vor allem Mahlen und Klassieren.

Auf dicken Glimmerplättchen basierende, mit Metalloxiden umhüllte Perlglanzpigmente haben aufgrund der Dicke des Randes einen deutlichen Streuanteil, speziell bei feineren Korngrößenverteilungen unter 20 μm.

Als Ersatz für Glimmer sind dünne Glasplättchen vorgeschlagen worden, die durch Walzen einer Glasschmelze mit nachfolgendem Mahlen erhalten werden. Interferenzpigmente auf der Basis derartiger Materialien weisen zwar Farbeffekte auf, die denen herkömmlicher, auf Glimmer basierender Pigmente überlegen sind. Nachteilig ist jedoch, daß die Glasplättchen eine sehr große mittlere Dicke von etwa 10-15 μm und eine sehr breite Dickenverteilung (typischerweise zwischen 4 und 20 μm) aufweisen, während die Dicke von Interferenzpigmenten typischerweise nicht größer als 3 μm ist.

In EP 0,384,596 wird ein Verfahren beschrieben, bei dem hydratisiertes Alkalisilikat bei Temperaturen von 480-500 °C mit einem Luftstrahl beaufschlagt wird, wobei sich Blasen mit dünnen Wandstärken bilden; die Blasen werden anschließend zerkleinert und man erhält plättchenförmige Alkalisilikatsubstrate mit einer Dicke von weniger als 3 μm. Das Verfahren ist jedoch aufwendig und die Dickenverteilung der erhaltenen Plättchen ist relativ breit.

In DE 11 36 042 ist ein kontinuierliches Bandverfahren zur Herstellung plättchen- oder flitterartiger Oxide oder Oxidhydrate von Metallen der IV. und V. Gruppe sowie der Eisen-Gruppe des Periodensystems beschrieben. Dabei wird auf ein kontinuierliches Band ggf. zunächst eine Trennschicht aus z.B. Silikonlack aufgebracht, um das spätere Ablösen der Metalloxidschicht zu erleichtern. Anschließend wird ein Flüssigkeitsfilm aus einer Lösung einer hydrolysierbaren Verbindung des in das gewünschte Oxid umzuwandelnden Metalls aufgebracht, der Film wird getrocknet und anschließend mit einer Rüttelvorrichtung abgelöst. Die Schichtdicke der erhaltenen Plättchen wird mit 0,2 bis 2 μm angegeben, ohne hierfür konkrete Beispiele zu nennen.

In EP 0,240,952 und EP 0,236,952 ist ein kontinuierliches Bandverfahren zur Herstellung verschiedener plättchenförmiger Materialien, darunter auch Siliciumdioxid, Aluminiumoxid und Titandioxid vorgeschlagen worden. Dabei wird über ein Rollensystem auf ein glattes Band ein dünner flüssiger Film definierter Dicke eines Precursors des plättchenförmigen Materials aufgebracht; der Film wird getrocknet und von dem Band abgelöst, wobei sich plättchenförmige Teilchen bilden. Die Teilchen werden anschließend gegebenenfalls geglüht, gemahlen und klassiert.

Die Dicke der nach dem in EP 0 240 952 beschriebenen Verfahren erhaltenen Plättchen ist relativ gut definiert, da der Film z.B. auf das kontinuierliche Band über ein Rollensystem sehr gleichmäßig aufgebracht wird. Die Schichtdicke der Plättchen wird in den Beispielen mit 0,3 bis 3,0 μm angegeben. Gemäß Bei-

spiel 1 wird eine erste Rolle mit dem verwendeten Precursor benetzt, indem man diese Rolle teilweise in einen mit dem Precursor befüllten Vorratsbehälter eintaucht. Der Film wird von dieser Rolle auf eine zweite, gleichsinnig rotierende Rolle übertragen, die mit der ersten in sehr engem Kontakt steht. Schließlich wird der Film von der zweiten Rolle auf das kontinuierliche Band abgerollt.

Nachteilig sind jedoch die Verwendung sehr teurer Precursormaterialien sowie insbesondere die erhöhten Anforderungen an die Arbeitsplatzsicherheit, die beim Einsatz metallorganischer Verbindungen gestellt werden müssen. Die vollständige chemische Umwandlung des Precursors in das gewünschte Schichtmaterial macht in der Regel eine starke Erhitzung des Filmes und des Bandmaterials erforderlich. Neben der dabei auftretenden erheblichen thermischen Belastung des Bandmaterials, wirken ' sich auch der hohe Energieaufwand und die Einschränkung der Prozeßgeschwindigkeit sehr nachteilig auf die Wirtschaftlichkeit des Verfahrens aus.

WO 93/08 237 beschreibt plättchenförmige Pigmente, bestehend aus einer plättchenförmigen Matrix aus Siliciumdioxid, die lösliche oder nichtlösliche Farbmittel enthalten kann und die mit einer oder mehreren reflektierenden Schichten aus Metalloxiden oder Metallen belegt ist. Die plättchenförmige Matrix wird durch Verfestigung und Hydrolyse von Wasserglas in einem endlosen Band hergestellt.

In DE 1 273 098 wird die Herstellung eines Perlmuttpigmentes durch Aufdampfen von ZnS-, $MgF_2$-, ZnO-, $CaF_2$- und $TiO_2$-Filmen auf ein endloses Band beschrieben. Dieses Verfahren ist aber ebenso, wie das in US 4 879140 beschriebene Verfahren, bei dem plättchenförmige Pigmente mit Si- und $SiO_2$-Schichten durch Plasmaabscheidung aus $SiH_4$ und $SiCl_4$ erhalten werden, mit einem sehr hohen apparativen Aufwand verbunden.

Aufgabe der Erfindung ist es, ein im wesentlichen transparentes Interferenzpigment mit kräftigen Interferenzfarben und/oder einer starken Winkelabhängigkeit der Interferenzfarben zur Verfügung zu stellen. Darüber hinaus ist es Aufgabe der Erfindung, ein Pigment bereitzustellen, das nur aus optisch funktionellen Schichten besteht und damit extrem dünn ist.

Diese Aufgabe wird gemäß der Erfindung gelöst durch ein mehrschichtiges Interferenzpigment, bestehend aus plättchenförmigem Titandioxid als Trägermaterial, das mit alternierenden Schichten von Metalloxiden mit niedriger und hoher Brechzahl beschichtet ist, wobei die Differenz der Brechzahlen mindestens 0,1 beträgt, erhältlich durch Verfestigung und Hydrolyse einer wäßrigen Lösung einer thermisch hydrolysierbaren anorganischen Titanverbindung auf einem endlosen Band, Ablösung der entstandenen Schicht, Beschichtung der erhaltenen Titandioxidplättchen nach oder ohne Zwischentrocknung im Naßverfahren abwechselnd mit einem Metalloxidhydrat mit hoher Brechzahl und einem Metalloxidhydrat mit niedriger Brechzahl durch Hydrolyse der entsprechenden, wasserlöslichen anorganischen Metallverbindungen, Abtrennung, Trocknung und gegebenenfalls Kalzination des erhaltenen Materials.

Weiterhin Wird diese Aufgabe gemäß der Erfindung gelöst durch ein Verfahren zur Herstellung des erfindungsgemäßen Pigmentes, wobei

- eine wäßrige Lösung einer thermisch hydrolysierbaren anorganischen Titanverbindung als dünner Film auf ein endloses Band aufgebracht wird,

- der flüssige Film durch Trocknung verfestigt wird und dabei das Titandioxid durch eine chemische Reaktion aus der Lösung entwickelt wird,

- die entstandene Schicht anschließend vom Band abgelöst und gewaschen wird,

- die erhaltenen Titandioxidplättchen nach oder ohne Zwischentrocknung in Wasser suspendiert und abwechselnd mit einem Metalloxidhydrat mit hoher Brechzahl und einem Metalloxidhydrat mit niedriger Brechzahl durch Zugabe und Hydrolyse der entsprechenden, wasserlöslichen anorganischen Metallverbindungen beschichtet werden und

- die beschichteten Titandioxidplättchen aus der wäßrigen Suspension abgetrennt, getrocknet und gegebenenfalls kalziniert werden.

Gegenstand der Erfindung ist außerdem die Verwendung der erfindungsgemäßen Pigmente zur Pigmentierung von Lacken, Druckfarben, Kunststoffen, Kosmetika, Glasuren für Keramiken und Gläser und zur Herstellung von Agrarfolien.

Hierfür können sie als Mischungen mit handelsüblichen Pigmenten, beispielsweise anorganischen und organischen Absorptionspigmenten, Metalleffektpigmenten und LCP-Pigmenten, eingesetzt werden.

Die erfindungsgemäßen Pigmente basieren auf plättchenförmigem Titandioxid. Diese Plättchen haben eine Dicke zwischen 10 nm und 500 nm, vorzugsweise zwischen 40 und 150 nm. Die Ausdehnung in die beiden anderen Dimensionen beträgt zwischen 2 und 200 µm und insbesondere zwischen 5 und 50 µm.

Bei dem Metalloxid mit hoher Brechzahl kann es sich um ein Oxid oder Mischungen von Oxiden mit oder ohne absorbierende Eigenschaften handeln, wie z.B. $TiO_2$, $ZrO_2$, $Fe_2O_3$, $Fe_3O_4$, $Cr_2O_3$ oder ZnO, oder um eine Verbindung mit hoher Brechzahl, wie z.B. Eisentitanate, Eisenoxidhydrate und Titansuboxide oder Mischungen bzw. Mischphasen dieser Verbindungen untereinander oder mit anderen Metalloxiden.

Das Metalloxid mit niedriger Brechzahl ist $SiO_2$, $Al_2O_3$, AIOOH, $B_2O_3$ oder eine Mischung daraus und kann ebenfalls absorbierende oder nicht absorbierende Eigenschaften haben. Gegebenenfalls kann die Oxidschicht mit niedriger Brechzahl Alkali- und Erdalkalioxide als Bestandteile enthalten.

Die Dicke der Schichten der Metalloxide mit hoher und niedriger Brechzahl ist wesentlich für die optischen Eigenschaften des Pigmentes. Da ein Produkt mit kräftigen Interferenzfarben erwünscht ist, muß die Dicke der Schichten aufeinander eingestellt werden. Wenn n die Brechzahl einer Schicht und d ihre Dicke ist, ergibt sich die Farbe, in der eine dünne Schicht erscheint, aus dem Produkt von n und d, d.h. der optischen Dicke. Die bei Normallichteinfall im reflektierten Licht entstehenden Farben eines solchen Filmes ergeben sich aus einer Verstärkung des Lichtes der Wellenlänge $\lambda = \frac{4}{2N-1} \cdot nd$ und durch Schwächung von Licht der Wellenlänge $\lambda = \frac{2}{N} \cdot nd$, worin N eine positive ganze Zahl ist. Die bei zunehmender Filmdicke erfolgende Variation der Farbe ergibt sich aus der Verstärkung bzw. Schwächung bestimmter Wellenlängen des Lichtes durch Interferenz. Beispielsweise hat ein 115 nm dicker Film aus Titandioxid mit der Brechzahl 1,94 eine optische Dicke von 115 x 1,94 = 223 nm, und Licht der Wellenlänge 2 x 223 nm = 446 nm (blau) wird bei der Reflexion geschwächt, so daß das reflektierte Licht gelb ist. Bei mehrschichtigen Pigmenten wird die Interferenzfarbe durch die Verstärkung bestimmter Wellenlängen bestimmt und wenn mehrere Schichten in einem vielschichtigen Pigment gleiche optische Dicke besitzen, wird die Farbe des reflektierten Lichtes mit zunehmender Zahl der Schichten intensiver und satter. Darüber hinaus kann durch geeignete Wahl der Schichtdicken eine besondere starke Variation der Farbe in Abhängigkeit vom Betrachtungswinkel erreicht werden. Es bildet sich ein ausgeprägter Farbflop aus, der für die Pigmente gemäß der Erfindung erwünscht sein kann. Die Dicke der einzelnen Metalloxidschichten, unabhängig von ihrer Brechzahl beträgt deshalb 20 bis 500 nm, vorzugsweise 50 bis 300 nm.

Die Anzahl und Dicke der Schichten ist abhängig vom gewünschten Effekt. Normalerweise erreicht man die gewünschten Effekte, wenn man das 5-Schichtsystem $TiO_2/SiO_2/TiO_2/SiO_2/TiO_2$ aufbaut und die Dicke der einzelnen Schichten optisch aufeinander abstimmt. Bei Verwendung relativ optisch dünnerer $TiO_2$-Schichten (Schichtdicke < 100 nm) lassen sich z.B. Interferenzpigmente mit blauer Farbe herstellen, die bei einem wesentlich geringeren $TiO_2$-Gehalt farbkräftiger und transparenter sind als reine $TiO_2$-Glimmerpigmente. Die Einsparung an $TiO_2$ beträgt bis zu 80 Gew:-%.

Durch die Auffällung dicker $SiO_2$-Schichten (Schichtdicke > 100 nm) werden Pigmente mit einer stark ausgeprägten Winkelabhängigkeit der Interferenzfarbe erhalten.

Durch Auffällen weiterer $SiO_2$- und $TiO_2$-Schichten lassen sich auch höhere Systeme erhalten, die Anzahl der Schichten wird aber dann durch die Wirtschaftlichkeit des Pigmentes begrenzt.

Da im Gegensatz zu Glimmer das plättchenförmige Titandioxid als Substrat eine optisch funktionelle Schicht ist, erhält man durch die Belegung des Substrates mit z.B. 2 Schichten des oben erwähnten Aufbaus ein Interferenzsystem aus 5 dünnen Schichten scharf definierter Dicken. Das Reflektions- bzw. Transmissionsspektrum eines solchen Pigmentes weist feinere und genauer abstimmbare Strukturen auf als das Spektrum eines entsprechenden Pigments, das auf einem Substrat mit breiter Dickenverteilung, z.B. Glimmer, beruht.

Diese Pigmente zeigen bereits mit extrem dünnen $TiO_2$-Schichten (Schichtdicke: 40 nm) kräftige Interferenzfarben. Besonders ausgeprägt ist auch die Winkelabhängigkeit der Interferenzfarbe. Dieser extreme Farbflop wird bei konventionellen Metalloxid-Glimmerpigmenten nicht beobachtet.

Die Herstellung der erfindungsgemäßen Pigmente erfolgt in einem zweistufigen Verfahren. In der ersten Stufe werden plättchenförmige Titandioxidpartikel mit Hilfe eines endlosen Bandes hergestellt.

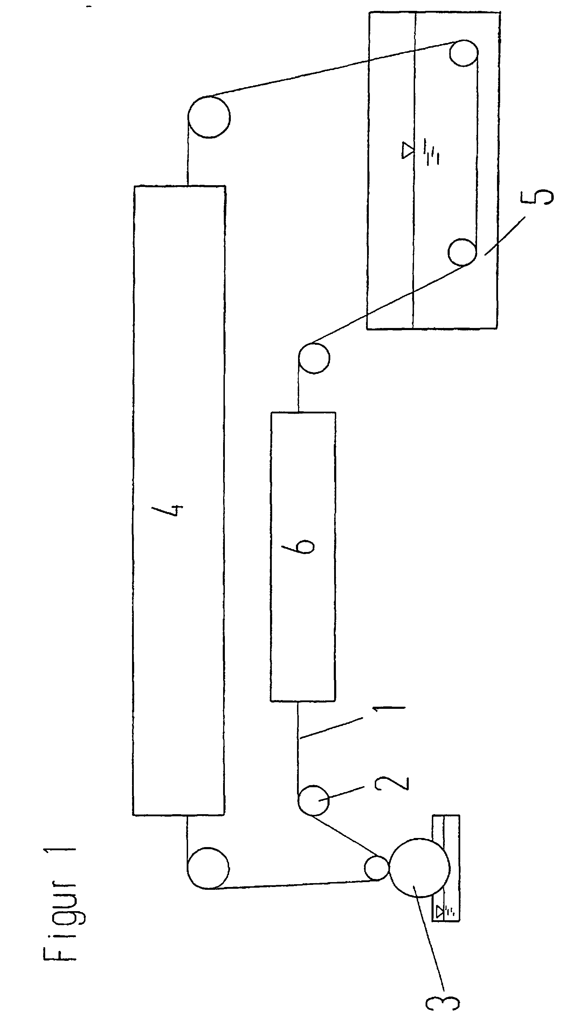

Zunächst soll an Hand von Figur 1 das Bandverfahren erläutert werden.

Das endlose Band 1, welches über ein Rollensystem 2 geführt wird, durchläuft ein Auftragswerk 3, wo es mit einem dünnen Film einer wäßrigen Lösung einer thermisch hydrolysierbaren anorganischen Titanverbindung beschichtet wird. Bevorzugt wird eine wäßrige Titantetrachloridlösung verwendet.

Die Konzentration des Titansalzes in diesen Lösungen beträgt 7 bis 30 Gew.-%, vorzugsweise 8 bis 15 Gew.-%. Ats geeignete Auftragswerke können Walzenauftragswerke sowie Fließer eingesetzt werden. Die Bandgeschwindigkeit liegt zwischen 2 und 400 m/min, vorzugsweise 5-200 m/min.

Um eine gleichmäßige Benetzung des Kunststoffbandes zu erzielen, ist es zweckmäßig, der Beschichtungslösung ein handelsübliches Netzmittel zuzusetzen oder die Bandoberfläche durch Beflammung, Koronabehandlung oder Ionisation zu aktivieren.

Anschließend durchläuft das beschichtete Band eine Trockenstrecke 4 in der die Schicht bei Temperaturen zwischen 30 und 200 °C getrocknet wird. Als Trockner können zum Beispiel handelsübliche Infrarot-, Umluftdüsen- und UV-Trockner eingesetzt werden.

Nach dem Passieren der Trockenstrecke wird das Band durch die Ablösebäder 5 mit einem geeignetem Ablösemedium, zum Beispiel vollentsalztem Wasser geführt, wo die getrocknete Schicht vom Band entfernt wird. Der Ablösevorgang wird hierbei durch zusätzliche Vorrichtungen, zum Beispiel Düsen, Bürsten oder Ultraschall, unterstützt.

In einem Nachtrockner 6 wird das Band vor der erneuten Beschichtung getrocknet.

Das endiose Band sollte aus einem chemisch und thermisch beständigem Kunststoff sein, um eine ausreichende Standzeit und hohe Trocknungstemperaturen zu gewährleisten. Hierfür sind Materialien wie Polyethytenterephthalat (PET) oder andere Polyester und Polyacrylate geeignet.

Die Folienbreite liegt typischerweise zwischen einigen Zentimetern bis zu mehreren Metern. Die Dicke beträgt zwischen 10 μm bis zu einigen mm, wobei diese beiden Parameter im Hinblick auf die jeweiligen Anforderungen optimiert werden.

Weitere Einzelheiten zu kontinuierlichen Bandverfahren sind aus US 3 138 475, EP 0 240 952 und WO 93/08 237 bekannt.

Die vom Band abgelösten Titandioxidplättchen werden in einer zweiten Stufe ohne vorherige Zwischentrocknung abwechselnd mit einem Metalloxidhydrat mit niedriger Brechzahl und einem Metalloxidhydrat mit hoher Brechzahl beschichtet.

Die Metalloxidschichten werden vorzugsweise naßchemisch aufgebracht, wobei die zur Herstellung von Perlglanzpigmenten entwickelten naßchemischen Beschichtungsverfahren angewendet werden können; derartige Verfahren sind z.B. beschrieben in DE 14 67 468, DE 19 59 988, DE 20 09 566, DE 22 14 545, DE 22 15 191, DE 22 44 298, DE 23 13 331 DE 25 22 572, DE 31 37 808, DE 31 37 809, DE 31 51 343, DE 31 51 354, DE 31 51 355, DE 32 11 602, DE 32 35 017 oder auch in weiteren Patentdokumenten und sonstigen Publikationen.

Bei der Naßbeschichtung werden die Substratpartikel in Wasser suspendiert und mit einem oder mehreren hydrolysierbaren Metallsalzen bei einem für die Hydrolyse geeigneten pH-Wert versetzt, der so gewählt wird, daß die Metalloxide bzw. Metalloxidhydrate direkt auf den Plättchen niedergeschlagen werden, ohne daß es zu Nebenfällungen kommt. Der pH-Wert wird üblicherweise durch gleichzeitiges Zudosieren einer Base konstant gehalten. Anschließend werden die Pigmente abgetrennt, gewaschen und getrocknet und gegebenenfalls geglüht, wobei die Glühtemperatur im Hinblick auf die jeweils vorliegende Beschichtung optimiert werden kann. Falls gewünscht können die Pigmente nach Aufbringen einzelner Beschichtungen abgetrennt, getrocknet und ggf. geglüht werden, um dann zur Auffällung der weiteren Schichten wieder resuspendiert zu werden.

Weiterhin kann die Beschichtung auch in einem Wirbelbettreaktor durch Gasphasenbeschichtung erfolgen, wobei z.B. die in EP 0,045,851 und EP 0,106,235 zur Herstellung von Perlglanzpigmenten vorgeschlagenen Verfahren entsprechend angewendet werden können.

Als Metalloxid mit hoher Brechzahl wird bevorzugt Titandioxid und als Metalloxid mit niedriger Brechzahl Siliziumdioxid verwendet.

Für das Aufbringen der Titandioxidschichten wird das im US 3 553 001 beschriebene Verfahren bevorzugt.

Zu einer auf etwa 50-100 °C, insbesondere 70-80 °C erhitzten Suspension des zu beschichtenden Materials wird langsam eine wäßrige Titansalzlösung zugegeben, und es wird durch gleichzeitiges Zudosieren einer Base, wie z.B. wäßrige Ammoniaklösung oder wäßrige Alkalilauge, ein weitgehend konstanter pH-Wert von etwa 0,5-5, insbesondere etwa 1,5-2,5 eingehalten. Sobald die gewünschte Schichtdicke der $TiO_2$-Fällung erreicht ist, wird die Zugabe der Titansalzlösung und der Base gestoppt.

Dieses, auch als Titrationsverfahren bezeichnete Verfahren zeichnet sich dadurch aus, daß ein Überschuß an Titansalz vermieden wird. Das wird dadurch erreicht, daß man pro Zeiteinheit nur eine solche Menge der Hydrolyse zuführt, wie sie für eine gleichmäßige Beschichtung mit dem hydratisierten $TiO_2$ erforderlich ist und wie pro Zeiteinheit von der verfügbaren Oberfläche der zu beschichtenden Teilchen aufgenommen werden kann. Es entstehen deshalb keine hydratisierten Titandioxidteilchen, die nicht auf der zu beschichtenden Oberfläche niedergeschlagen sind.

Für das Aufbringen der Siliziumdioxidschichten ist folgendes Verfahren anzuwenden: Zu einer auf etwa 50-100 °C, insbesondere 70-80 °C erhitzten Suspension des zu beschichtenden Materials wird eine Natronwasserglaslösung zudosiert. Durch gleichzeitige Zugabe von 10%iger Salzsäure wird der pH-Wert bei 7,5 konstant gehalten. Sobald die gewünschte Schichtdicke der $SiO_2$-Fällung erreicht ist, wird die Zugabe der Wasserglaslösung gestoppt. Anschließend wird nach 30 min nachgerührt.

Die naßchemische Erzeugung mehrerer hoch und niedrig brechender Interferenzschichten mit genau definierter Dicke und glatter Oberfläche auf feinteiligen, plättchenförmigen Substraten ist bisher nicht bekannt. Zu beachten ist, daß auf das transparente Trägermaterial zuerst ein Metalloxid mit niedriger Brechzahl aufgebracht werden muß.

Es ist weiterhin möglich, das fertige Pigment einer Nachbeschichtung oder Nachbehandlung zu unterziehen, die die Licht-, Wetter- und chemische Stabilität weiter erhöht, oder die Handhabung des Pigments, insbesondere die Einarbeitung in unterschiedliche Medien, erleichtert. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage.

Die zusätzlich aufgebrachten Stoffe machen nur etwa 0,1 bis 5 Gew.-%, vorzugsweise etwa 0,5 bis 3 Gew.-%, des gesamten Pigmentes aus.

Das erfindungsgemäße Pigment kann noch zusätzlich mit schwerlöslichen, fest haftenden anorganischen oder organischen Farbmitteln beschichtet werden. Bevorzugt werden Farblacke und insbesondere Aluminiumfarblacke verwendet. Dazu wird eine Aluminiumhydroxidschicht aufgefällt, die in einem zweiten Schritt mit einem Farblack verlackt wird. Das Verfahren ist in DE 24 29 762 und DE 29 28 287 näher beschrieben.

Bevorzugt ist auch eine zusätzliche Beschichtung mit Komplexsalzpigmenten, insbesondere Cyanoferratkomplexen, wie zum Beispiel Berliner Blau und Turnbulls Blau, wie sie in EP 0 141 173 und DE 23 13 332 beschrieben ist.

Das erfindungsgemäße Pigment kann auch mit organischen Farbstoffen und insbesondere mit Phthalocyanin- oder Metallphthalocyanin- und/oder Indanthrenfarbstoffen nach DE 40 09 567 beschichtet werden. Dazu wird eine Suspension des Pigmentes in einer Lösung des Farbstoffes hergestellt und diese dann mit einem Lösungsmittel zusammengebracht, in welchem der Farbstoff schwer löslich oder unlöslich ist.

Weiterhin können auch Metallchalkogenide bzw. Metalchalkogenidhydrate und Ruß für eine zusätzliche Beschichtung eingesetzt werden.

Die folgenden Beispiele sollen die Erfindung näher erläutern, ohne sie zu begrenzen.

**Beispiel 1**

Ein umlaufendes Band aus Polyethylenterephthalat (Breite: 0,3 m, Geschwindigkeit: 20 m/min) wird über eine Auftragswalze im Gegenlauf mit einer 20%iger Titantetrachloridlösung beschichtet. Die Beschichtungslösung enthält 0,3 Gew.-% Tensid (DISPERSE-AYD W-28, Hersteller: DANIEL PRODUCTS COMPANY). Der auf dem Band befindliche wäßrige Film wird in einer Trocknungsstrecke durch Beaufschlagung mit Heißluft von 70 °C getrocknet und die gebildete Schicht in einem mit vollentsalztem Wasser gefüllten Ablösebecken vom Band abgelöst. Die Titandioxidplättchen werden filtriert und mit vollentsatztem Wasser gewaschen. Die silbrig glänzenden Ptättchen haben eine Schichtdicke von $100 \pm 10$ nm.

Zur weiteren Beschichtung werden sie entweder in volfentsalztem Wasser redispergiert oder bei 110 °C getrocknet.

**Beispiel 2**

**5-Schicht-System aus $TiO_2$ / $SiO_2$ / $TiO_2$ / $SiO_2$ / $TiO_2$**

1) $SiO_2$-Schicht:
 50 g $TiO_2$-Flakes mit gelber Interferenzfarbe (Teilchengröße < 20 $\mu$m) werden in 1,5 l vollentsalztem Wasser suspendiert und auf 75 °C erhitzt. Zu dieser Suspension werden innerhalb von 90 min 270 ml einer Natronwasserglas-Lösung (125 g $SiO_2$/l) bei 75 °C zudosiert. Dabei wird der pH-Wert mit 10%iger Salzsäure konstant auf 7,5 gehalten. Nach beendeter Zugabe wird zur Vervollständigung der Fällung 30 min bei 75 °C nachgerührt.

2) $TiO_2$-Schicht
 Der pH-Wert der Suspension wird mit 10%iger Salzsäure auf 2,2 abgesenkt und innerhalb von 3 h werden 590 ml einer wäßrigen $TiCl_4$-Lösung (400 g $TiCl_4$/l) hinzudosiert. Der pH-Wert wird während der gesamten Zugabe mit 32%iger NaOH-Lösung bei 2,2 konstant gehalten. Nach beendeter Zugabe wird zur Vervollständigung der Fällung noch 30 min bei 75 °C nachgerührt.

 Anschließend läßt man auf Raumtemperatur abkühlen, filtriert das erhaltene Pigment ab, wäscht mit vollentsalztem Wasser salzfrei und trocknet bei 110 °C. Danach wird das Pigment 30 min bei 700 °C geglüht. Das so erhaltene Pigment zeigt eine leuchtende, goldene Interferenzfarbe, die wesentlich intensiver als die des Ausgangsmaterials ist.

**Beispiel 3**

**5-Schicht-System aus $TiO_2$ / $SiO_2$ / $TiO_2$ / $SiO_2$ / $TiO_2$ mit blauer Interferenzfarbe**

1) $SiO_2$-Schicht:
 40 g $TiO_2$-Flakes mit silberner Interferenzfarbe werden in 1,5 l VE-Wasser suspendiert und auf 75 °C erhitzt. Zu dieser Suspension werden innerhalb von 180 min 580 ml einer Natronwasserglas-Lösung (125 g $SiO_2$/l) bei 75 °C zudosiert. Dabei wird der pH-Wert mit 10%iger Salzsäure konstant auf 7,5 gehalten. Nach beendeter Zugabe wird zur Vervollständigung der Fällung 30 min bei 75 °C nachgerührt.

2) $TiO_2$-Schicht
 Der pH-Wert der Suspension wird mit 10%iger Salzsäure auf 2,2 abgesenkt und innerhalb von 120 min werden 470 ml einer wäßrigen $TiCl_4$-Lösung (400 g $TiCl_4$/l) hinzudosiert. Der pH-Wert wird während der gesamten Zugabe mit 32%iger NaOH-Lösung bei 2,2 konstant gehalten. Nach beendeter Zugabe wird zur Vervollständigung der Fällung noch 30 min bei 75 °C nachgerührt.

 Anschließend läßt man auf Raumtemperatur abkühlen, filtriert das erhaltene Pigment ab, wäscht mit VE-Wasser salzfrei und trocknet bei 110 °C. Danach wird das Pigment 30 min bei 700 °C geglüht.

 Das so erhaltene Pigment zeigt eine tiefblaue Interferenzfarbe.

**Patentansprüche**

1. Mehrschichtiges Interferenzpigment, bestehend aus plättchenförmigem Titandioxid als Trägermaterial, das mit altemierenden Schichten von Metalloxiden mit niedriger und hoher Brechzahl beschichtet ist, wobei die Differenz der Brechzahlen mindestens 0,1 beträgt, erhältlich durch Verfestigung und Hydrolyse einer wäßrigen Lösung einer thermisch hydrolysierbaren anorganischen Titanverbindung auf einem endlosen Band, Ablösung der entstandenen Schicht, Beschichtung der erhaltenen Titandioxidplättchen nach oder ohne Zwischentrocknung im Naßverfahren abwechselnd mit einem Metalloxidhydrat mit hoher Brechzahl und einem Metalloxidhydrat mit niedriger Brechzahl durch Hydrolyse der entsprechenden, wasserlöslichen anorganischen Metallverbindungen, Abtrennung, Trocknung und gegebenenfalls Kalzination des erhaltenen Materials.

2.   Interferenzpigment nach Anspruch 1, **dadurch gekennzeichnet, daß** das Oxid mit hoher Brechzahl TiO$_2$, ZrO$_2$, Fe$_2$O$_3$, Fe$_3$O$_4$, Cr$_2$O$_3$, ZnO oder ein Gemisch aus diesen Oxiden oder ein Eisentitanat, ein Eisenoxidhydrat, ein Titansuboxid oder eine Mischung bzw. Mischphase dieser Verbindungen ist.

3.   Interferenzpigment nach den Ansprüchen 1 und 2, **dadurch gekennzeichnet, daß** das Metalloxid mit niedriger Brechzahl SiO$_2$, Al$_2$O$_3$, AlOOH, B$_2$O$_3$ oder eine Mischung daraus ist, wobei gegebenenfalls Alkali- und Erdalkalioxide als zusätzliche Bestandteile enthalten sein können.

4.   Verfahren zur Herstellung des erfindungsgemäßen Pigmentes nach den Ansprüchen 1 bis 3, **dadurch gekennzeichnet, daß**

-   eine wäßrige Lösung einer thermisch hydrolysierbaren anorganischen Titanverbindung als dünner Film auf ein endloses Band aufgebracht wird,

-   der flüssige Film durch Trocknung verfestigt wird und dabei das Titandioxid durch eine chemische Reaktion aus der Lösung entwickelt wird,

-   die entstandene Schicht anschließend vom Band abgelöst und gewaschen wird,

-   die erhaltenen Titandioxidplättchen nach oder ohne Zwischentrocknung in Wasser suspendiert und abwechselnd mit einem Metalloxidhydrat mit hoher Brechzahl und einem Metalloxidhydrat mit niedriger Brechzahl durch Zugabe und Hydrolyse der entsprechenden, wasserlöslichen anorganischen Metallverbindungen beschichtet werden und

-   die beschichteten Titandioxidplättchen aus der wäßrigen Suspension abgetrennt, getrocknet und gegebenenfalls kalziniert werden.

5.   Verfahren nach Anspruch 4, **dadurch gekennzeichnet, daß** das Oxid mit hoher Brechzahl TiO$_2$, ZrO$_2$, Fe$_2$O$_3$, Fe$_3$O$_4$, Cr$_2$O$_3$, ZnO oder ein Gemisch aus diesen Oxiden oder ein Eisentitanat, ein Eisenoxidhydrat, ein Titansuboxid oder eine Mischung bzw. Mischphase dieser Verbindungen ist.

6.   Verfahren nach Anspruch 4, **dadurch gekennzeichnet, daß** das Metalloxid mit niedriger Brechzahl SiO$_2$, Al$_2$O$_3$, AlOOH, B$_2$O$_3$ oder eine Mischung daraus ist, wobei gegebenenfalls Alkali- und Erdalkalioxide als zusätzliche Bestandteile enthalten sein können.

7.   Verfahren nach mindestens einem der Ansprüche 4 bis 6, **dadurch gekennzeichnet, daß** die Metalloxide nach Zwischentrocknung des zu beschichtenden Materials in einem Wirbelbettreaktor durch CVD aufgebracht werden.

8.   Verwendung der Pigmente nach den Ansprüchen 1 bis 3 zur Pigmentierung von Lacken, Druckfarben, Kunststoffen, Kosmetika, Agrarfolien und Glasuren für Keramiken und Gläser.

9.   Verwendung nach Anspruch 8, **dadurch gekennzeichnet, daß** die Pigmente als Mischungen mit handelsüblichen Pigmenten eingesetzt werden.

10.  Lacke, Druckfarben, Kunststoffe, Kosmetika, Agrarfolien, Keramiken und Gläser, welche mit einem Pigment nach den Ansprüchen 1 und 3 pigmentiert sind.

**Claims**

1.   Multilayer interference pigment consisting of plateletlike titanium dioxide as carrier material, coated with alternating layers of metal oxides of low and high refractive index, the difference in the refractive indices being at least 0.1, which is obtainable by solidification and hydrolysis of an aqueous solution of a thermally hydrolysable inorganic titanium compound on a continuous belt, detachment of the resulting coat, coating of the resulting titanium dioxide platelets, with or without drying in between, by a wet method with, alternately, a metal oxide hydrate of high refractive index and a metal oxide hydrate of low refractive index by hydrolysis of the corresponding, water-soluble inorganic metal compounds, separation, drying and, if desired, calcining of the material obtained.

2.   Interference pigment according to Claim 1, **characterized in that** the oxide of high refractive index is TiO$_2$, ZrO$_2$, Fe$_2$O$_3$, Fe$_3$O$_4$, Cr$_2$O$_3$, ZnO or a mixture of these oxides or an iron titanate, an iron oxide hydrate, a titanium suboxide or a mixture or mixed phase of these compounds.

3.   Interference pigment according to Claims 1 and 2, **characterized in that** the metal oxide of low refrac-

tive index is $SiO_2$, $Al_2O_3$, AlOOH, $B_2O_3$ or a mixture thereof, it being possible if desired for alkali metal oxides and alkaline earth metal oxides to be present as additional constituents.

4. Process for the preparation of the novel pigment according to Claims 1 to 3, **characterized in that**

   - an aqueous solution of a thermally hydrolysable inorganic titanium compound is applied as a thin film to a continuous belt,
   - the liquid film is solidified by drying, during the course of which the titanium dioxide is developed from the solution by means of a chemical reaction,
   - the resulting layer is subsequently detached from the belt and washed,
   - the titanium dioxide platelets obtained, with or without drying in between, are suspended in water and coated with, alternately, a metal oxide hydrate of high refractive index and a metal oxide hydrate of low refractive index, by addition and hydrolysis of the corresponding, water-soluble inorganic metal compounds, and
   - the coated titanium dioxide platelets are separated out from the aqueous suspension, dried and, if desired, calcined.

5. Process according to Claim 4, **characterized in that** the oxide of high refractive index is $TiO_2$, $ZrO_2$, $Fe_2O_3$, $Fe_3O_4$, $Cr_2O_3$, ZnO or a mixture of these oxides or an iron titanate, an iron oxide hydrate, a titanium suboxide or a mixture or mixed phase of these compounds.

6. Process according to Claim 4, **characterized in that** the metal oxide of low refractive index is $SiO_2$, $Al_2O_3$, AlOOH, $B_2O_3$ or a mixture thereof, it being possible if desired for alkali metal oxides and alkaline earth metal oxides to be present as additional constituents.

7. Process according to at least one of Claims 4 to 6, **characterized in that** the metal oxides are applied in a fluidized-bed reactor by CVD following intermediate drying of the material to be coated.

8. Use of the pigments according to Claims 1 to 3 for pigmenting paints, printing inks, plastics, cosmetics, agricultural films and glazes for ceramics and glass.

9. Use according to Claim 8, **characterized in that** the pigments are employed as mixtures with commercially available pigments.

10. Paints, printing inks, plastics, cosmetics, agricultural films, ceramics and glass which are pigmented with a pigment according to Claims 1 to 3.

**Revendications**

1. Pigment d'interférence multicouche, constitué de dioxyde de titane lamellaire en tant que matériau support, qui est revêtu de couches alternées d'oxydes métalliques ayant un faible indice de réfraction et un grand indice de réfraction, la différence entre les indices de réfraction étant d'au moins 0,1, que l'on peut obtenir par solidification et hydrolyse d'une solution aqueuse d'un composé du titane inorganique thermiquement hydrolysable sur une bande sans fin, détachement de la couche obtenue, application sur les plaquettes de dioxyde de titane obtenues, après ou sans séchage intermédiaire, par un procédé par voie humide, en alternance d'un oxyhydroxyde métallique ayant un grand indice de réfraction et d'un oxyhydroxyde métallique ayant un faible indice de réfraction, par hydrolyse des composés métalliques inorganiques solubles dans l'eau correspondants, séparation, séchage et éventuellement calcination du matériau obtenu.

2. Pigment d'interférence selon la revendication 1, **caractérisé en ce que** l'oxyde ayant un grand indice de réfraction est $TiO_2$, $ZrO_2$, $Fe_2O_3$, $Fe_3O_4$, $Cr_2O_3$, ZnO ou un mélange de ces oxydes, ou un titanate de fer, un oxyhydroxyde de fer, un sous-oxyde de titane ou un mélange ou une phase mixte de ces composés.

3. Pigment d'interférence selon les revendications 1 et 2, **caractérisé en ce que** l'oxyde métallique ayant un faible indice de réfraction est $SiO_2$, $Al_2O_3$, AlOOH, $B_2O_3$ ou un mélange de ceux-ci, auquel cas éventuellement des oxydes de métaux alcalins et alcalino-terreux peuvent être présents en tant que constituants additionnels.

4. Procédé de fabrication du pigment selon l'invention selon les revendications 1 à 3, **caractérisé en ce que**

   - on applique sur une bande sans fin, sous forme d'un film mince, une solution aqueuse d'un composé du titane inorganique thermiquement hydrolysable,
   - on solidifie le film liquide par séchage, ce à l'occasion de quoi lc dioxydc de titane se dégage de la solution par une réaction chimique,
   - puis on détache de la bande la couche obtenue, et on la lave,
   - après ou sans séchage intermédiaire, on met en suspension dans de l'eau les plaquettes de dioxyde de titane obtenues, et on les revêt en alternance d'un oxyhydroxyde métallique ayant un grand indice de réfraction et d'un oxyhydroxyde métallique ayant un faible indice de réfraction, par addition et hydrolyse des composés métalliques inorganiques solubles dans l'eau correspondants, et

- on sépare de la suspension aqueuse les plaquettes de dioxyde de titane revêtues, on les sèche et éventuellement on les calcine.

5. Procédé selon la revendication 4, **caractérisé en ce que** l'oxyde ayant un grand indice de réfraction, est $TiO_2$, $ZrO_2$, $Fe_2O_3$, $Fe_3O_4$, $Cr_2O_3$, $ZnO$ ou un mélange de ces oxydes, ou un titanate de fer, un oxyhydroxyde de fer, un sous-oxyde de titane ou un mélange ou une phase mixte de ces composés.

6. Procédé selon la revendication 4, **caractérisé en ce que** l'oxyde métallique ayant un faible indice de réfraction est $SiO_2$, $Al_2O_3$, $AlOOH$, $B_2O_3$ ou un mélange de ceux-ci, auquel cas éventuellement des oxydes de métaux alcalins et alcalino-terreux peuvent être présents en tant que constituants additionnels.

7. Procédé selon au moins l'une des revendications 4 à 6, **caractérisé en ce que** les oxydes métalliques sont, après séchage intermédiaire du matériau devant être revêtu dans un réacteur à lit fluidisé, appliqués par CVD.

8. Utilisation des pigments selon les revendications 1 à 3 pour pigmenter des vernis, des encres d'imprimerie, des plastiques, des cosmétiques, des feuilles à usage agricole et des glaçures pour céramiques et verres.

9. Utilisation selon la revendication 8, **caractérisée en ce que** les pigments sont utilisés sous forme de mélanges avec des pigments du commerce.

10. Vernis, encres d'imprimerie, plastiques, produits cosmétiques, feuillés à usage agricole, céramiques et verres, qui sont pigmentés par un pigment selon les revendications 1 à 3.

Figur 1

EP 0 944 677 B1

4

6

2

1

3

5